# EUROPEAN PATENT APPLICATION

(11) **EP 4 159 223 A1**
(43) Date of publication of application: **05.04.2023**
(21) Application number: 21816672.6
(22) Date of filing: 01.06.2021
(51) Int. Cl.: A61K 35/32, A61P 25/28, A23L 33/10, G01N 33/68, G01N 33/50

(54) **COMPOSITION COMPRISING OSTEOPONTIN INHIBITOR AS ACTIVE INGREDIENT FOR PREVENTION, ALLEVIATION, OR TREATMENT OF NEURODEGENERATIVE DISEASE**

(30) Priority: 02.06.2020 KR 20200066649; 31.05.2021 KR 20210069644
(71) Applicant: The Catholic University Of Korea Industry-Academic Cooperation Foundation, Seocho-gu Seoul 06591 (KR)
(72) Inventor: KIM, Sung Won, Seoul 06001 (KR); PARK, Soon A, Seoul 03013 (KR); LEE, Jung Eun, Suwon-si, Gyeonggi-do 16336 (KR); JEUN, Sin-Soo, Seoul 06701 (KR); LIM, Jung Yeon, Seoul 06090 (KR); YANG, Seung Ho, Seoul 06284 (KR)
(74) Representative: Dehns
(86) International application number: PCT/KR2021/006768
(87) International publication number: WO 2021/246744

(57) **Abstract**

The present invention relates to a pharmaceutical composition comprising an agent inhibiting gene expression or protein activity of osteopontin as an active ingredient for preventing or treating neurodegenerative disease. According to the present invention, there is an effect of suppressing amyloid beta (Aβ)-induced neuronal cell death by downregulating the expression or activity of osteopontin. In addition, an inhibitor against the expression or activity of osteopontin decreases a level of pro-inflammatory proteins and conversely, increases a level of anti-inflammatory proteins. Therefore, it is expected that the present invention can be advantageously used as a therapeutic agent for various neurodegenerative diseases including Alzheimer's disease.

## Description

### [Technical Field]

The present invention relates to a composition for preventing, improving or treating a neurodegenerative disease, which includes an agent for inhibiting osteopontin gene expression or osteopontin protein activity as an active ingredient.

This application claims priority to and the benefit of Korean Patent Application No. 10-2020-0066649, filed on June 2, 2020, and Korean Patent Application No. 10-2021-0069644, filed on May 31, 2021, and the disclosure of which is incorporated herein by reference in their entireties.

### [Background Art]

A neurodegenerative disease refers to a disease that occurs in the brain or spinal cord among degenerative diseases that occur with aging, and a disease that is caused by the gradual loss of structure and function of specific brain cell groups in the brain and spinal cord due to an unknown cause, a genetic defect or an environmental factor. Neuronal cell death or damage caused by the neurodegenerative disease is known to cause problems in the formation or function of synapses that deliver information between brain neuronal cells, which are most critical for the information delivery of the brain nervous system, and an ideal increase or decrease in electrical activity of brain nerves. Neuronal cells in the brain and spinal cord have very diverse functions depending on a location, causing characteristic dysfunction due to the damage to neuronal cells in a specific region, and show a wide variety of clinical aspects depending on what type of functional disorder progresses. These neurodegenerative diseases include Alzheimer's disease (AD), Parkinson's disease (PD), Huntington's disease (HD), multiple sclerosis (MS), and amyotrophic lateral sclerosis (ALS). Neurodegenerative diseases including Alzheimer's disease are mainly known by the accumulation of amyloid beta (Aβ), and studies have been focused on removing plaques formed by aggregation of this protein.

Meanwhile, osteopontin is a phosphoprotein including an intrinsic arginine-glycine-aspartic acid (Arg-Gly-Asp; RGD) cell-binding sequence, which was first isolated from rat osteosarcoma (ROS 17/2.8) cells. OPN is secreted from osteoblasts and osteoclasts to act as a type of bridge (Latin pons) and plays a role in attaching cells to the matrix. However, many researchers have confirmed that osteopontin is expressed in various tissues, such as the kidneys, inner ear, arterial smooth muscle, digestive tract epithelium, neuronal cell body and axons in addition to bone, and present an opinion that its function also vary depending on an expression region.

In relation to the nervous system, osteopontin has been reported to have neuroprotective and anti-inflammatory effects in various types of diseases such as subarachnoid hemorrhage, stroke, multiple sclerosis, and Parkinson's disease, and conversely, there are reports that a peptide consisting of 6 amino acids containing the RGD motif of osteopontin has no neuroprotective effect or acts as a competitive inhibitor of osteopontin. As such, osteopontin appears to have opposite effects of causing neurotoxicity and death in some situations in a general neurodegenerative disease and acting as a neuroprotective agent in other circumstances. The inventors confirmed a phenomenon in which the expression of osteopontin is reduced while studying the mechanism of action of a stem cell therapeutic in an Alzheimer's disease model, and completed the present invention.

### [Disclosure]

### [Technical Problem]

Therefore, the inventors confirmed that neurodegenerative diseases including Alzheimer's disease can be effectively treated by inhibiting the expression or activity of osteopontin (OPN), and thus the present invention was completed.

The present invention is directed to providing a pharmaceutical composition for preventing or treating a neurodegenerative disease, which includes an agent for inhibiting OPN gene expression or OPN protein activity as an active ingredient.

The present invention is also directed to providing a health functional food composition for preventing or improving a neurodegenerative disease, which includes the above agent as an active ingredient.

The present invention is also directed to providing a method of screening a material for preventing, improving or treating a neurodegenerative disease.

However, technical problems to be solved in the present invention are not limited to the above-described problems, and other problems which are not described herein will be fully understood by those of ordinary skill in the art belonging to the present invention from the following descriptions.

### [Technical Solution]

To achieve the purposes of the present invention, the present invention provides a pharmaceutical composition for preventing or treating a neurodegenerative disease, which includes an agent inhibiting OPN gene expression or OPN protein activity as an active ingredient.

The present invention also provides a health functional food composition for preventing or improving a neurodegenerative disease, which includes an agent inhibiting OPN gene expression or OPN protein activity as an active ingredient.

The present invention also provides a method of preventing, improving, or treating a neurodegenerative disease, which includes administering a pharmaceutical composition including an agent inhibiting OPN gene expression or OPN protein activity as an active ingredient into a subject in need thereof.

In addition, the present invention provides a use of a pharmaceutical composition including an agent inhibiting OPN gene expression or OPN protein activity as an active ingredient for prevention, improvement or treatment of a neurodegenerative disease.

In addition, the present invention provides a use of a pharmaceutical composition including an agent inhibiting OPN gene expression or OPN protein activity as an active ingredient for preparation of a drug for preventing or treating a neurodegenerative disease.

In one embodiment of the present invention, the OPN may consist of an amino acid sequence represented by SEQ ID NO: 1, but the present invention is not limited thereto.

In another embodiment of the present invention, the agent for inhibiting OPN gene expression may be selected from the group consisting of miRNA, siRNA, shRNA, a ribozyme, a DNAzyme, a peptide nucleic acid (PNA) and an antisense oligonucleotide, complementarily binding to mRNA of the OPN gene, but the present invention is not limited thereto.

In still another embodiment of the present invention, the agent for inhibiting OPN protein activity may be selected from the group consisting of a peptide, an antibody, an aptamer and a compound, which specifically bind to OPN protein, but the present invention is not limited thereto.

In yet another embodiment of the present invention, the neurodegenerative disease may be selected from the group consisting of Alzheimer's disease, Parkinson's disease, Huntington's disease, multiple sclerosis, amyotrophic lateral sclerosis and dementia, but the present invention is not limited thereto.

In yet another embodiment of the present invention, the composition may inhibit the death of neuronal cells caused by amyloid beta (Aβ).

In yet another embodiment of the present invention, the composition may decrease the number of immune-inflammatory cells or the level of a pro-inflammatory protein, or increase the number of an anti-inflammatory protein, but the present invention is not limited thereto.

In yet another embodiment of the present invention, the pro-inflammatory protein may be interleukin-6 (IL-6), and the anti-inflammatory protein may be interleukin-10 (IL-10), but the present invention is not limited thereto.

In addition, the present invention provides a method of screening a material for preventing, improving or treating a neurodegenerative disease, which includes (a) bringing a candidate material into contact with an isolated cell or tissue; (b) measuring an expression level of OPN protein in the cell or tissue; and (c) determining the candidate material as a material for preventing, improving or treating a neurodegenerative disease when the expression level of OPN protein in the cell or tissue contacted with the candidate material is reduced compared to the level before contact with the candidate material.

In one embodiment of the present invention, the isolated cell or tissue may be a neuronal cell or nerve tissue, but the present invention is not limited thereto.

### [Advantageous Effects]

According to the present invention, the death of neuronal cells caused by amyloid beta (Aβ) can be inhibited by inhibiting the expression or activity of osteopontin, the level of a pro-inflammatory protein can be reduced, and in contrast, the level of an anti-inflammatory protein can increase. Therefore, it is expected that the present invention can be effectively used as a therapeutic for various neurodegenerative diseases including Alzheimer's disease.

### [Description of Drawings]

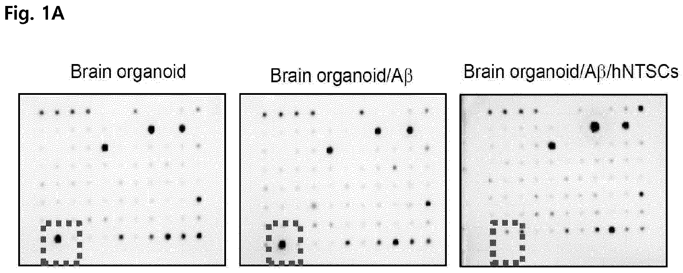

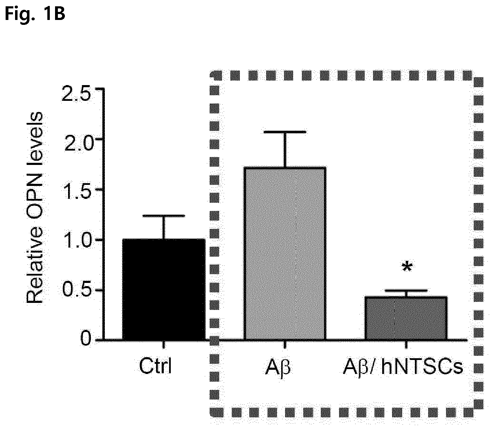

FIGS. 1A and 1B are results of the cytokine array (FIG. 1A) and the quantitative analysis of an osteopontin protein expression level (FIG. 1B), in a brain organoid culture after three-dimensional (3D) brain organoids are treated with amyloid beta (Aβ) oligomers or DMSO alone as a control, or co-treated with amyloid beta (Aβ) oligomers and human nasal inferior turbinate-derived stem cells (amyloid beta (Aβ); human nasal inferior turbinate-derived stem cells (hNTSCs); osteopontin (OPN)).

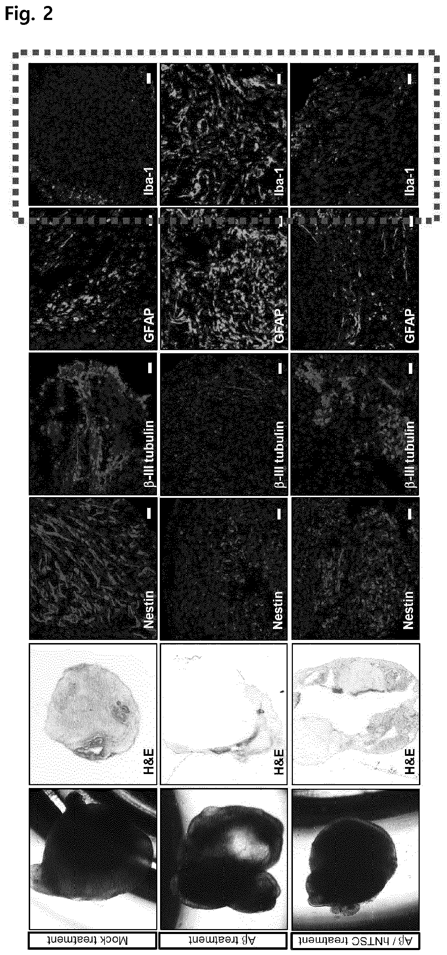

FIG. 2 shows the result of immunofluorescence staining for the expression of nestin, class III β-tubulin, GFAP and OPN expression-related inflammatory cell marker Iba-1 after treatment of human nasal inferior turbinate-derived stem cells in an Alzheimer's disease model using three-dimensional brain organoids.

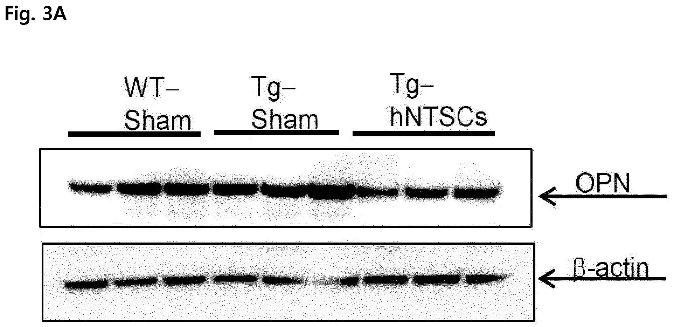

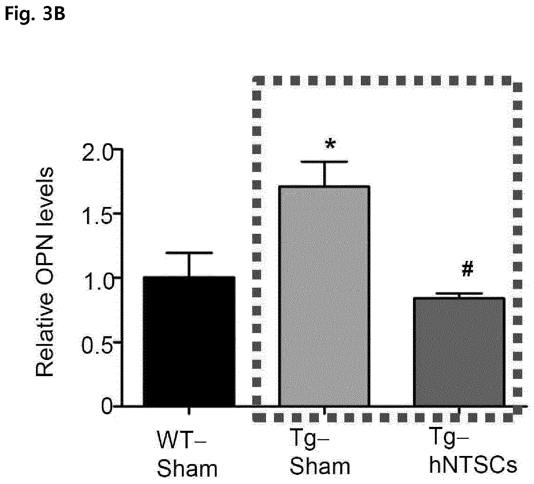

FIGS. 3A and 3B are the result of western blotting (FIG. 3A) confirming an OPN expression level in animal brain after human nasal inferior turbinate-derived stem cell transplantation in an Alzheimer's disease animal model and a graph quantifying the same (FIG. 3B).

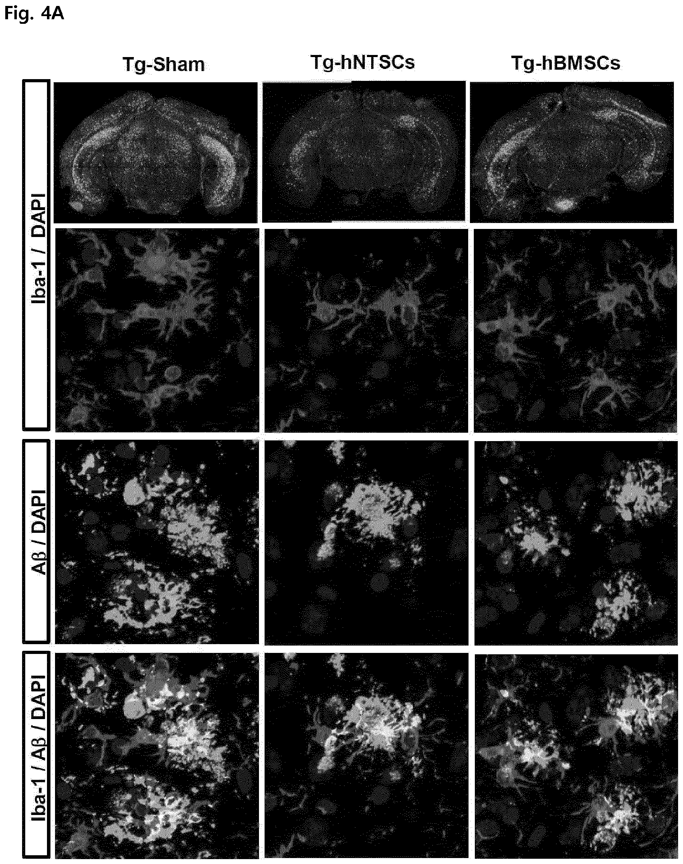

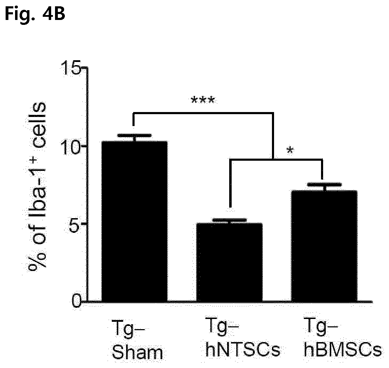

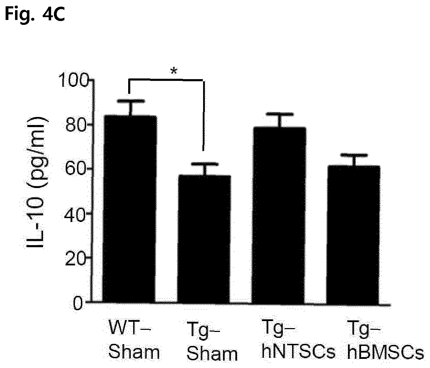

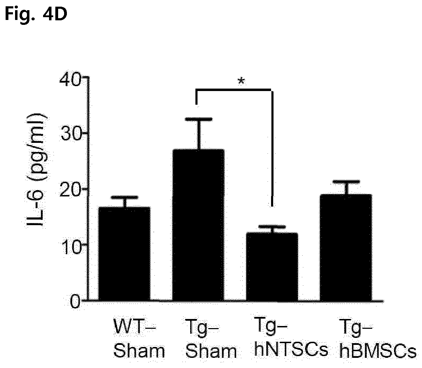

FIGS. 4A to 4D are the results of confirming the expression level of inflammatory cell marker Iba-1 in animal brains after human nasal inferior turbinate-derived stem cell transplantation in Alzheimer's disease animal models using immunofluorescence staining (FIGS. 4A and 4B) and graphs quantified by confirming expression levels of anti-inflammatory proteins IL-10 and IL-6 using ELISA (FIGS. 4C and 4D).

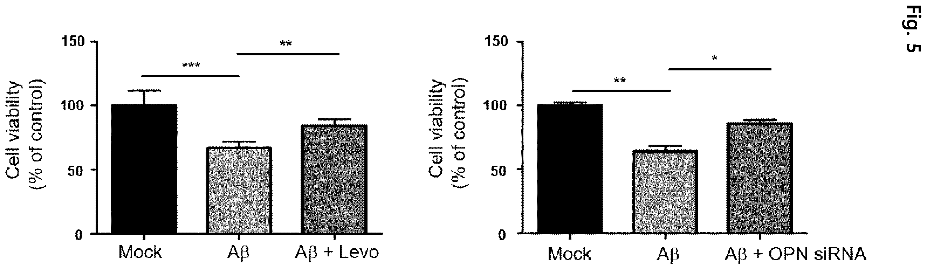

FIG. 5 shows the result of evaluating amyloid beta toxicity levels according to OPN-specific antagonist and siRNA treatments using an MTT assay.

### [Modes of the Invention]

The present invention may provide a pharmaceutical composition for preventing or treating a neurodegenerative disease, which includes an agent for inhibiting osteopontin gene expression or osteopontin protein activity as an active ingredient.

As another aspect of the present invention, the present invention may provide a method of preventing, improving or treating a neurodegenerative disease, which includes administering the above composition into a subject in need thereof.

The term "osteopontin (OPN)" used herein refers to an integrin- and calcium-binding phosphoprotein including an intrinsic arginine-glycine-aspartic acid (Arg-Gly-Asp; RGD) cell-binding sequence, and is known to be produced in calcified tissue, various epithelial cells, and cells of an activated immune system and found in the kidneys, pancreas, urinary/reproductive system, lungs, salivary glands, inner ear, nervous system, placenta and urine. OPN has CD44- and integrin-based receptors, and interacts with the receptors to participate in various physiological processes such as cell adhesion, cell migration, signal transduction, and inflammation.

In the present invention, OPN may be human-derived OPN, and may consist of, for example, an amino acid sequence represented by SEQ ID NO: 1, but the present invention is not limited thereto.

In the present invention, the agent for inhibiting OPN gene expression may be selected from the group consisting of miRNA, siRNA, shRNA, a ribozyme, a DNAzyme, a peptide nucleic acid (PNA) and an antisense oligonucleotide, complementarily binding to mRNA of the OPN gene.

The term "miRNA, siRNA and shRNA" used herein refers to nucleic acid molecules that mainly bind to mRNA transcribed from a target gene to mediate RNA interference or gene silencing, thereby suppressing the translation of the mRNA. The miRNA, siRNA and shRNA may be used in an effective gene knockdown method or gene therapy method because they can inhibit the expression of a target gene at the translation level.

The term "siRNA" used herein may have a double-stranded structure in which a sense strand (e.g., a sequence corresponding to the OPN gene mRNA sequence) and an antisense strand (e.g., a sequence complementary to the OPN gene mRNA sequence) are located opposite each other. In addition, the siRNA molecule that can be used in the present invention may have a single-stranded structure having self-complementary sense and antisense strands. siRNA is not limited to the complete pairing of double-stranded RNA parts that pair with each other, and may include parts that do achieve pairing due to a mismatch (the corresponding bases are not complementary) or a bulge (there is no base corresponding to one chain). Specifically, the total length is 10 to 100 bases, more specifically, 15 to 80 bases, and even more specifically 20 to 70 bases.

The term "small hairpin RNA or short hairpin RNA (shRNA)" used herein refers to sequences of RNAthat makes a strong hairpin turn, and may be used to silence gene expression by RNA interference. shRNA may be introduced into cells using any promoter that can function in eukaryotic cells. Such a vector is always transferred to daughter cells so that gene silencing can be inherited. The shRNA hairpin structure is degraded into siRNA, an intracellular machinery, and bound to an RNA-induced silencing complex. The above-described complex binds to siRNA-matched mRNA and degrades it. shRNA is transcribed by RNA polymerase III, and shRNA production in mammalian cells may cause an interferon response like when cells recognize shRNA as a viral attack and seek a defense mechanism.

The term "microRNA (miRNA)" used herein refers to a single-stranded RNA molecule of 21 to 25 nucleotides, and a material for controlling gene expression in a eukaryote by binding to the 3"-UTR of messenger RNA (mRNA) (BartelDP, et al., Cell, 23;116(2): 281-297(2004)). miRNA is produced as precursor miRNA (pre-miRNA) with a stem-roof structure by RNaseIII type enzyme (Drosha), transferred to the cytoplasm, cleaved by a dicer, and then formed into mature miRNA.

The term "ribozyme" used herein is a catalytic RNAmolecule that can degrade a nucleic acid molecule having a completely or partially complementary sequence with respect to the ribozyme sequence. A ribozyme transgene encoding an RNA ribozyme that specifically pairs with target RNA and functionally inactivates target RNA by degrading a phosphodiester backbone at a specific location may be designed. During such degradation, the ribozyme itself is not changed, and thus may be recycled to degrade a different molecule. The ribozyme may be directly targeted to cells in the form of an RNA oligonucleotide into which the ribozyme sequence is incorporated, or may be introduced into cells as an expression vector encoding desired ribozyme RNA. The ribozyme may be used and applied in substantially the same manner as described for an antisense oligonucleotide.

The term "DNAzyme" used herein is a catalytic DNA molecule that cleaves single-stranded RNA, is highly selective for a target RNA sequence and thus can be used to downregulate a specific gene by targeting mRNA.

The term "peptide nucleic acid (PNA)" used herein refers to an artificially synthesized polymer similar to DNA or RNA, and was first introduced in 1991 by Professors Nielsen, Egholm, Berg and Buchardt of the University of Copenhagen, Denmark. While DNA has a phosphate-ribose backbone, a PNA has a repeated N-(2-aminoethyl)-glycine backbone linked by peptide bonds, thereby greatly increasing binding strength and stability to DNA or RNA, and thus is used in molecular biology, diagnostic analysis and antisense therapies. PNA is disclosed in detail in the literature [Nielsen PE, Egholm M, Berg RH, Buchardt O (December 1991), "Sequence-selective recognition of DNA by strand displacement with a thymine-substituted polyamide", Science 254(5037): 1497-1500].

The term "antisense oligonucleotide" used herein refers to DNA or RNA containing a nucleic acid sequence complementary to a specific mRNA sequence, or a derivative thereof, and may bind to a complementary sequence in OPN mRNA to inhibit mRNA translation into a protein.

In the present invention, the agent for inhibiting OPN protein activity may be selected from the group consisting of a peptide, an antibody, an aptamer and a compound, specifically binding to the OPN protein.

The term "antibody" used herein refers to a protein material that can specifically bind to an antigenic site of a protein or peptide molecule. The type of antibody is not particularly limited, and a part thereof may also be included as long as it is a polyclonal antibody, a monoclonal antibody or any one that has antigen binding ability, and may include not only all immunoglobulin antibodies, but also special antibodies such as a humanized antibody. Moreover, the antibody includes a complete form with two full-length light chains and two full-length heavy chains, and a functional fragment of the antibody molecule. A functional fragment of the antibody molecule refers to a fragment possessing at least an antigen-binding function, and may include Fab, F(ab'), F(ab')₂, Fv, and etc.

The term "aptamer" used herein refers to a single-stranded oligonucleotide having a size of approximately 20 to 60 nucleotides, which is a type of polynucleotide having a stabilized 3D structure as it is and consists of a single-stranded nucleic acid (DNA, RNA or modified nucleic acid), characterized by binding to a target molecule with high affinity and specificity. The aptamer may use various materials such as a polynucleotide, a polypeptide, a compound and a polymer as a target molecule, and has higher stability than a protein, and is easily synthesized due to a simple structure and consisting of a nucleic acid, and therefore it is used in methods of detecting various molecular molecules. The aptamer may bind to a predetermined target molecule, thereby inhibiting the activity of the predetermined target molecule. The aptamer may be RNA, DNA, a modified nucleic acid, or a mixture thereof, and may be in a linear or cyclic form.

In the present invention, the compound may be any one that is known as an OPN inhibitor in the art, but the present invention is not limited thereto, and may be, for example, levocetirizine. The levocetirizine may be represented by Formula 1 below, but the present invention is not limited thereto.

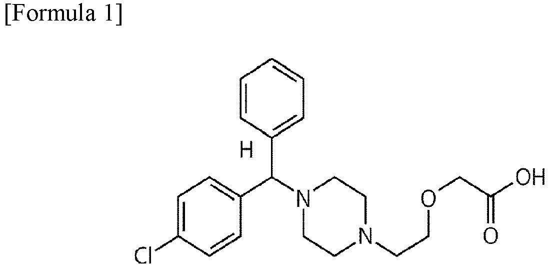

In addition, the compound of the present invention includes not only a pharmaceutically acceptable salt, but also all salts, isomers, hydrates and solvates, which can be prepared by conventional methods.

The term "neurodegenerative disease" used herein refers to a condition or disorder that cause damage, dysfunction or complications that are characterized by deterioration of cognitive function, or neurological, neurodegenerative, biological, mental or behavioral aberrations since neuronal cells are lost due to apoptosis. Suitable neurodegenerative diseases that can be prevented or treated with the pharmaceutical composition of the present invention may include Alzheimer's disease, Parkinson's disease, Huntington's disease, multiple sclerosis, amyotrophic lateral sclerosis and dementia, but the present invention is not limited thereto.

In addition, in the present invention, the composition may inhibit neuronal cell death caused by amyloid beta (Aβ).

The term "amyloid beta (Aβ)" may be interchangeably used with "Abeta" or "Aβ," and refers to the major chemical components found in the brain and spinal cord of patients with various neurodegenerative diseases Alzheimer's disease. Amyloid beta (Aβ) is a fragment of amyloid precursor protein (APP) including various numbers of amino acids, generally, 38 to 43 amino acids.

In the present invention, the composition may have an effect of reducing the number of immune inflammatory cells.

In addition, in the present invention, the composition may have an effect of reducing the level of a pro-inflammatory protein or increasing the level of an anti-inflammatory protein. Here, the pro-inflammatory protein may be interleukin-6 (IL-6), and the anti-inflammatory protein may be interleukin-10 (IL-10), but the present invention is not limited thereto.

In one embodiment of the present invention, in *in vitro* Alzheimer's disease models using amyloid beta (Aβ) and three-dimensional brain organoids, when human nasal inferior turbinate-derived stem cells are co-cultured, it was confirmed that OPN expression significantly decreased, which is caused by a significant decrease in the number of inflammatory cells producing OPN (see Examples 1 and 2).

In addition, in one embodiment of the present invention, when human nasal inferior turbinate-derived stem cells are transplanted into Alzheimer's disease animal models overexpressing amyloid beta (Aβ), it was confirmed that OPN protein expression was highly inhibited, the number of inflammatory cells producing OPN was greatly reduced, and an expression level of the anti-inflammatory protein interleukin-10 increased, whereas the expression of the pro-inflammatory protein interleukin-6 greatly decreased (see Examples 3 and 4).

In addition, in one embodiment of the present invention, when neural stem cells were treated with levocetirizine or OPN siRNA with amyloid beta, it was confirmed that apoptosis caused by amyloid beta was greatly reduced (see Example 6).

Meanwhile, a content of the active ingredient in the composition of the present invention can be suitably adjusted according to a disease symptom, the progression of the symptom, or the condition of a patient, and for example, may be 0.0001 to 99.9 wt%, or 0.001 to 50 wt% with respect to the total weight of the composition, but the present invention is not limited thereto. The content ratio is a value based on a dry content without a solvent.

The pharmaceutical composition of the present invention may further include suitable carrier, excipient and diluent, which are conventionally used in preparation of a pharmaceutical composition. The excipient may be one or more selected from the group consisting of, for example, a diluent, a binder, a disintegrant, a glidant, an adsorbent, a humectant, a film-coating material, and a controlled release additive.

The pharmaceutical composition according to the present invention may be formulated in the form of a powder, a granule, a sustained-release granule, an enteric granule, a solution and a liquid, an ophthalmic solution, an elixir, an emulsion, a suspension, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a capsule, a perfusate, a plaster, a lotion, a paste, a spray, an inhalant, a patch, a sterile injection, or an external preparation such as an aerosol according to a conventional method, and the external preparation may be formulated in a cream, a gel, a patch, a spray, an ointment, a plaster, a lotion, a liniment, a paste or a cataplasma.

The carrier, excipient and diluent which may be included in the pharmaceutical composition according to the present invention may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil.

The composition may be formulated using a diluent or an excipient such as a filler, an extending agent, a binder, a wetting agent, a disintegrant, a surfactant, which are conventionally used.

As additives for a tablet, powder, granule, capsule, pill and troche, excipients such as corn starch, potato starch, wheat starch, lactose, sucrose, glucose, fructose, di-mannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose (HPMC), HPMC 1928, HPMC 2208, HPMC 2906, HPMC 2910, propylene glycol, casein, calcium lactate and Primojel; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution and light anhydrous silicic acid; and lubricants such as calcium stearate, magnesium stearate, stearic acid, hydrogenated vegetable oil, talc, limestone kaolin, petrolatum, sodium stearate, cacao butter, sodium salicylate, magnesium salicylate, polyethylene glycol 4000 and, 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dl-leucine and light anhydrous silicic acid may be used.

As an additive for the liquid according to the present invention, water, diluted hydrochloric acid, diluted sulfuric acid, sodium citrate, sucrose monostearate, polyoxyethylene sorbitol fatty acid ester (Tween ester), polyoxyethylene monoalkyl ether, lanolin ether, lanolin ester, acetic acid, hydrochloric acid, aqueous ammonia, ammonium carbonate, potassium hydroxide, sodium hydroxide, prolamine, polyvinylpyrrolidone, ethyl cellulose, or sodium carboxymethylcellulose may be used.

In the syrup according to the present invention, a sucrose solution, other sugars or sweeteners may be used, and if necessary, a fragrance, a colorant, a preservative, a stabilizer, a suspending agent, an emulsifier, or a viscous preparation may be used.

In the emulsion according to the present invention, distilled water may be used, and if necessary, an emulsifier, a preservative, a stabilizer or a fragrance may be used.

In the suspension according to the present invention, a suspending agent such as acacia, tragacanth, methylcellulose, carboxymethylcellulose, sodium carboxymethylcellulose, microcrystalline cellulose, sodium alginate, hydroxypropylmethylcellulose (HPMC), HPMC 1828, HPMC 2906, or HPMC 2910 may be used, and if necessary, a surfactant, a preservative, a stabilizer, a colorant, and a fragrance may be used.

In the injection according to the present invention, a solvent such as injectable sterile water, 0.9% sodium chloride for injection, Ringer's solution, dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing agent such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gums; an isotonic agent such as sodium chloride; a stabilizer such as sodium bisulfite (NaHSO₃), carbon dioxide gas, sodium metabisulfite (Na₂S₂O₃), sodium sulfite (Na₂SO₃), nitrogen gas (N₂) or ethylenediaminetetracetic acid; an antioxidant such as sodium bisulfide 0.1%, sodium formaldehyde sulfoxylate, thiourea, disodium ethylenediaminetetraacetate or acetone sodium bisulfite; a pain-relief agent such as benzyl alcohol, chlorobutanol, procaine hydrochloride, glucose or calcium gluconate; or a suspending agent such as sodium CMC, sodium alginate, Tween 80 or aluminum monostearate, may be used.

In the suppository according to the present invention, a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium, A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or a Tegester triglyceride base (TG-95, MA, 57) may be used.

A solid formulation for oral administration may be a tablet, a pill, a powder, a granule or a capsule, and prepared by mixing at least one or more excipients, for example, starch, calcium carbonate, sucrose, lactose and gelatin. In addition, in addition to simple excipients, lubricants such as magnesium stearate and talc may also be used.

A liquid formulation for oral administration may be a suspension, a liquid for internal use, an emulsion or a syrup, and a generally-used simple diluent such as water or liquid paraffin, as well as various types of excipients, for example, a wetting agent, a sweetener, a fragrance and a preservative may be included. A formulation for parenteral administration may be a sterilized aqueous solution, a non-aqueous solvent, a suspension, an emulsion, a lyophilized preparation or a suppository. As the non-aqueous solvent or suspension, propylene glycol, polyethylene glycol, a vegetable oil such as olive oil, or an injectable ester such as ethyl oleate may be used.

The pharmaceutical composition may be administered at a pharmaceutically effective amount. The term "pharmaceutically effective amount" used herein refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art.

The pharmaceutical composition of the present invention may be administered into a subject via various routes. All administration routes may be expected, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined according to the type of drug as an active ingredient along with several related parameters such as a disease to be treated, the route of administration, a patient's age, sex, weight, and the severity of the disease.

The term "subject" used herein refers to a target in need of treatment, and more specifically, a mammal such as a human or a non-human primate, a mouse, a rat, a dog, a cat, a horse and a cow.

The "administration" used herein refers to providing the composition of the present invention to a subject by a suitable method.

The term "prevention" used herein refers to all actions of inhibiting or delaying the onset of a target disease, the term "treatment" used herein refers to all actions involved in improving or beneficially changing symptoms of a target disease and a metabolic abnormalities caused thereby by administration of the pharmaceutical composition according to the present invention, and the term "improvement" used herein refers to all actions of reducing the degrees of parameters associated with a target disease, for example, the degree of symptoms, by administration of the composition according to the present invention.

As still another aspect of the present invention, the present invention may provide a health functional food composition for preventing or improving a neurodegenerative disease, which includes an agent for inhibiting OPN gene expression or OPN protein activity as an active ingredient.

When the agent of the present invention is used as a food additive, the agent may be added as it is or used with a different food or food ingredient, and may be suitably used according to a conventional method. The mixing ratio of the active ingredient may be properly determined depending on a purpose (prophylactic, health or therapeutic treatment). In general, in the production of food or beverages, the agent may be added in an amount of 15 wt% or less, or 10 wt% or less based on the raw material. However, in the case of long-term intake for health and hygiene or health control, the amount may be less than the above range, and since there is no problem in terms of safety, the active ingredient may be used in an amount larger than the above range.

There is no particular limit to the type of food. Examples of food to which the material is added may include meat, sausage, bread, chocolate, candy, snacks, confectioneries, pizza, ramen, other noodles, gum, dairy products including ice cream, various soups, beverages, tea, drinks, alcoholic beverages and vitamin complexes, and include all health functional foods in the ordinary sense.

The health beverage composition according to the present invention may contain additional components such as various flavoring agents or natural carbohydrates like conventional beverages. The above-mentioned natural carbohydrates are monosaccharides such as glucose and fructose, disaccharides such as maltose and sucrose, polysaccharides such as dextrin and cyclodextrin, and sugar alcohols such as xylitol, sorbitol and erythritol. As the sweetener, natural sweeteners such as thaumatin and a stevia extract, or synthetic sweeteners such as saccharin and aspartame may be used. The proportion of the natural carbohydrate may generally be approximately 0.01 to 0.20 g or 0.04 to 0.10 g per 100 mL of the composition of the present invention.

In addition, the composition of the present invention may contain various nutrients, vitamins, electrolytes, flavoring agents, stain, pectic acid and a salt thereof, alginic acid and a salt thereof, organic acids, protective colloidal thickening agents, pH adjustors, stabilizers, preservatives, glycerin, alcohols, or carbonizing agents used in carbonated beverages. Other than these, the composition of the present invention may contain fruit pulp for producing natural fruit juices, fruit drinks and vegetable drinks. Such components may be used independently or in combination. The proportion of such an additive is not very critical, but is generally selected in the range of 0.01 to 0.20 parts by weight per 100 parts by weight of the composition of the present invention.

As yet another aspect of the present invention, the present invention may provide a method of screening a material for preventing, improving or treating a neurodegenerative disease, which includes (a) bringing a candidate material into contact with an isolated cell or tissue; (b) measuring an expression level of OPN protein in the cell or tissue; and (c) determining the candidate material as a material for preventing, improving or treating a neurodegenerative disease when the expression level of OPN protein in the cell or tissue contacted with the candidate material is reduced compared to the level before contact with the candidate material.

In the present invention, the isolated cells or tissue may be neuronal cells or nerve tissue.

The method of measuring a level of the protein includes western blotting, enzyme linked immunosorbent assay (ELISA), radioimmunoassay (RIA), radioimmunodiffusion, Ouchterlony immunodiffusion, Rocket immunoelectrophoresis, tissue immunostaining, immunoprecipitation assay, complement fixation assay, fluorescence-activated cell sorting (FACS) and protein chip, but the present invention is not limited thereto.

Terms and words used in the specification and claims should not be construed as being limited to general or dictionary terms meanings, and should be interpreted with the meaning and concept in accordance with the technical idea of the present invention based on the principle that the inventors have appropriately defined the concepts of terms in order to explain the present invention in the best way.

Hereinafter, to help in understanding the present invention, exemplary examples will be suggested. However, the following examples are merely provided to more easily understand the present invention, and not to limit the present invention.

### [Examples]

### Example 1. Confirmation of change in OPN expression by human nasal inferior turbinate-derived stem cells in Alzheimer's disease model using amyloid beta and 3D brain organoids

The inventors confirmed a change in expression of cytokines and proteins by human nasal inferior turbinate-derived stem cells (hNTSCs) in *in vitro* Alzheimer's disease models.

Specifically, 9 × 10³ or 1 × 10⁴ dedifferentiated stem cells induced from human blood were mixed with mTeSR^{™} medium and then seeded in a U-bottom 96-well plate, and cultured in an incubator at 37 °C in a 5% CO₂ environment for approximately 7 days to induce spheroids. Next, the formed spheroids were differentiated using a differentiation medium containing an N2 supplement for approximately 5 to 7 days. Three-dimensional brain organoids were manufactured by incubating the cells in a shaker in an incubator at a speed of 70 rpm, 37 °C and in a 5% CO₂ environment for approximately 30 to 40 days while changing the medium at approximately 3- or 4-day intervals until becoming a complete mature body. After treating the organoid-cultured plate with 10 µM amyloid beta (Aβ) oligomers, in a Transwell system, an upper chamber in which hNTSCs were plated as an experimental group, and an upper chamber in which only a medium was added as a control were co-cultured for 3 days. Afterward, each culture (supernatant) was collected, and the cytokine array and a protein expression level were quantified and analyzed.

As a result, as shown in FIGS. 1A and 1B, compared with the control, it was confirmed that the OPN expression level significantly increases in the brain organoids treated with the amyloid beta (Aβ) oligomers alone, whereas in the group co-cultured with hNTSCs after the amyloid beta (Aβ) oligomer treatment, the OPN expression level is significantly reduced. The above result shows the therapeutic effect by stem cells is likely to appear by reducing OPN expression in the Alzheimer's disease model.

### Example 2. Confirmation of change in expression of neuronal cells and immune inflammatory cells by hNTSCs in Alzheimer's disease models using amyloid beta and 3D brain organoids

After the three-dimensional brain organoids manufactured by the same method as in Example 1 were treated with 10 µM amyloid beta (Aβ) oligomers, in a Transwell system, an upper chamber in which hNTSCs were plated as an experimental group, and an upper chamber in which only a medium was added as a control were co-cultured for 3 days. After the culture, the brain organoids were washed once with 1X phosphate-buffered saline (PBS), fixed with 4% paraformaldehyde for 24 hours, treated with a 15% or 30% sucrose solution. Afterward, a block was formed using an optimal cutting temperature (OCT) compound, and a frozen slide was prepared. The slide was washed with PBS and then permeabilized by treatment with 0.1% Triton X-100. It was washed again with PBS, treated with 1% normal goat serum (NGS) at room temperature for 1 hour to block non-specific binding, and then treated with a primary antibody (nestin, beta-tubulin III, GFAP or Iba-1) against a neuronal cell-related protein to perform a reaction at 4 °C for 24 hours. Afterward, the resultant was washed with PBS, treated with a fluorescence-conjugated secondary antibody against the primary antibody (Alexa Fluor 594-conjugates), and reacted at room temperature for 1 hour. The reaction product was washed with PBS, mounted with a mounting solution, and the expression of a neuronal cell-related protein was analyzed by immunofluorescence staining using confocal microscopy. 4',6-diamidino-2-phenylindole (DAPI) was used for counterstaining.

As a result, as shown in FIG. 2, compared with the control, it was confirmed that in the brain organoids treated with amyloid beta (Aβ) oligomers alone, the number of cells expressing an inflammatory cell marker Iba-1 was significantly increased, whereas in the group co-cultured with hNTSCs after treatment with the amyloid beta (Aβ) oligomers, the number of Iba-1-expressing cells was significantly decreased. The above results show that the number of OPN-expressing inflammatory cells is decreased, and reveal that a decrease in OPN expression is caused by a decrease in the number of inflammatory cells in terms of the Alzheimer's disease treatment effect by stem cells.

### Example 3. Confirmation of change in OPN expression according to transplantation of hNTSCs in Alzheimer's disease animal model overexpressing amyloid beta

To confirm whether the aspects of the therapeutic effect by stem cells confirmed in the *in vitro* Alzheimer's disease models in Examples 1 and 2 are shown in the same manner *in vivo,* the inventors confirmed the therapeutic effect and OPN expression level by transplantation of hNTSCs into Alzheimer's disease animal models.

As a result, as shown in FIGS. 3A and 3B, it was confirmed that OPN protein expression in the hNTSC-treated group was greatly inhibited. This result is consistent with the result of reducing OPN expression according to the therapeutic effect of stem cells confirmed in Examples 1 and 2.

### Example 4. Confirmation of changes in expression of immune inflammatory cells and inflammatory protein according to transplantation of hNTSCs in Alzheimer's disease animal model overexpressing amyloid beta

Next, the inventors confirmed the expression of immune inflammatory cells, the level of a pro-inflammatory protein and the level of an anti-inflammatory protein according to hNTSC treatment using an Alzheimer's disease animal model.

As a result, as shown in FIGS. 4A and 4B, it was confirmed that the expression of the immune inflammatory cell marker, Iba-1, was greatly inhibited in the hNTSC-treated animal model. In addition, as shown in FIGS. 4C and 4D, it was confirmed that the expression level of the anti-inflammatory protein, interleukin-10 (IL-10), was increased in the hNTSC-treated group, whereas the expression of the pro-inflammatory protein, interleukin-6 (IL-6), was greatly reduced. The above results prove that the levels of the immune inflammatory cells and the pro-inflammatory protein are reduced by a decrease in OPN expression according to the transplantation effect of stem cells, and the decrease in OPN expression according to the present invention is a critical factor for proving the therapeutic effect of the stem cells.

### Example 5. Confirmation of Alzheimer's disease treatment effect according to inhibition of OPN expression

The inventors confirmed an Alzheimer's disease treatment effect of the stem cells by the decrease in OPN according to Examples 1 to 4, and confirmed whether a direct decrease in the OPN level also shows the same therapeutic effect on Alzheimer's disease. It was confirmed that the therapeutic effect on Alzheimer's disease was exhibited when OPN protein expression was inhibited by targeting an OPN-encoding gene or mRNA transcribed therefrom was inhibited, or OPN protein activity was directly inhibited.

### Example 6. Confirmation of change in amyloid beta neurotoxicity according to inhibition of OPN expression in neuronal cells

Next, to confirm the function of the OPN protein for amyloid beta neurotoxicity, the inventors confirmed the level of amyloid beta toxicity according to the treatment of an OPN antagonist or OPN siRNA using neuronal cells.

Specifically, approximately 5 × 10⁴ neural stem cells were seeded in a 24-well plate, and cultured at 37 °C in a CO₂ incubator for 24 hours. The medium in the cell-containing plate was replaced with an antibiotic-free, 0.1% serum-containing medium, treated with 0.5 µM levocetirizine (Sigma-Aldrich, L7795, OPN antagonist), and after 6 to 7 hours, the cells were further treated with 3 µM amyloid beta, and after 48 hours of culture, cell viability was confirmed through an MTT assay.

In addition, in the case of an siRNA experiment, 2 × 10⁵ cells per well were seeded in an FBS-containing, antibiotic-free normal growth medium in a 6-well culture plate and cultured at 37 °C in a CO₂ incubator for 24 hours. The cells were treated with an OPN siRNA (Santacruz Biotechnology, sc-36129) mixture using a transfection reagent and incubated for approximately 6 to 7 hours at 37 °C in a CO₂ incubator. After culture, 1 ml of normal culture medium was further added and further cultured for approximately 24 hours. After culture, the medium in the plate was replaced with an antibiotic-free, 0.1% serum-containing medium, and the resultant cells were treated with 3 µM amyloid beta, and after about 24 hours of culture, cell viability was confirmed through an MTT assay.

As a result, as shown in FIG. 5, it was confirmed that, when neural stem cells were treated with 3 µM amyloid beta, the cell viability was approximately 60%, but when treated with 0.5 µM levocetirizine or 80 pmol OPN siRNA as well as amyloid beta, apoptosis caused by amyloid beta greatly decreased.

The above results show that the levels of the immune inflammatory cells and the pro-inflammatory protein are reduced by the decrease or inhibition of OPN expression, proving that the inhibition of OPN according to the present invention can be a treatment target of a neurodegenerative disease.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

According to the present invention, the death of neuronal cells caused by amyloid beta (Aβ) can be inhibited by inhibiting the expression or activity of osteopontin, the level of a pro-inflammatory protein can be reduced, and in contrast, the level of an anti-inflammatory protein can increase. Therefore, it is expected that the present invention can be effectively used as a therapeutic for various neurodegenerative diseases including Alzheimer's disease, and thus has industrial availability.

## Claims

1. A pharmaceutical composition for preventing or treating a neurodegenerative disease, comprising an agent for inhibiting osteopontin gene expression or osteopontin protein activity as an active ingredient.

2. The pharmaceutical composition of claim 1, wherein the osteopontin consists of an amino acid sequence represented by SEQ ID NO: 1.

3. The pharmaceutical composition of claim 1, wherein the agent for inhibiting osteopontin gene expression is selected from the group consisting of miRNA, siRNA, shRNA, a ribozyme, a DNAzyme, a peptide nucleic acid (PNA) and an antisense oligonucleotide, complementarily binding to mRNA of the osteopontin gene.

4. The pharmaceutical composition of claim 1, wherein the agent for inhibiting osteopontin protein activity is selected from the group consisting of a peptide, an antibody, an aptamer and a compound, specifically binding to the osteopontin protein.

5. The pharmaceutical composition of claim 1, wherein the neurodegenerative disease is selected from the group consisting of Alzheimer's disease, Parkinson's disease, Huntington's disease, multiple sclerosis, amyotrophic lateral sclerosis and dementia.

6. The pharmaceutical composition of claim 1, wherein the composition inhibits the death of neuronal cells caused by amyloid beta (Aβ).

7. The pharmaceutical composition of claim 1, wherein the composition reduces the number of immune inflammatory cells or the level of a pro-inflammatory protein, or increases the level of an anti-inflammatory protein.

8. The pharmaceutical composition of claim 7, wherein the pro-inflammatory protein is interleukin-6 (IL-6), and the anti-inflammatory protein is interleukin-10 (IL-10).

9. A health functional food composition for preventing or improving a neurodegenerative disease, comprising an agent for inhibiting osteopontin gene expression or osteopontin protein activity as an active ingredient.

10. A method of screening a material for preventing, improving or treating a neurodegenerative disease, comprising the following steps:
(a) bringing a candidate material into contact with an isolated cell or tissue;
(b) measuring an expression level of osteopontin protein in the cell or tissue; and
(c) determining the candidate material as a material for preventing, improving or treating a neurodegenerative disease when the expression level of osteopontin protein in the cell or tissue contacted with the candidate material is reduced compared to the level before contact with the candidate material.

11. The method of claim 10, wherein the isolated cell or tissue is a neuronal cell or nerve tissue.

12. A method of preventing or treating a neurodegenerative disease, comprising:
administering a pharmaceutical composition comprising an agent for inhibiting osteopontin gene expression or osteopontin protein activity as an active ingredient into a subject in need thereof.

13. A use of a pharmaceutical composition comprising an agent for inhibiting osteopontin gene expression or osteopontin protein activity as an active ingredient for preventing, improving or treating a neurodegenerative disease.

14. A use of a pharmaceutical composition comprising an agent for inhibiting osteopontin gene expression or osteopontin protein activity as an active ingredient for preparing a drug for preventing or treating a neurodegenerative disease.
